# EUROPEAN PATENT SPECIFICATION

(11) **EP 2 941 385 B1**
(45) Date of publication and mention of the grant of the patent: **11.12.2019**
(21) Application number: 13866684.7
(22) Date of filing: 26.12.2013
(51) Int. Cl.: A61J 1/20, B65B 3/04

(54) **NEEDLE KIT**
NADELSET
KIT POUR AIGUILLE

(30) Priority: 27.12.2012 US 201261746190 P
(43) Date of publication of application: 11.11.2015
(73) Proprietor: Medi-Physics, Inc., Princeton, NJ 08540 (US)
(72) Inventor: HELLE, Kevin M., Bartlett, Illinois 60103 (US)
(74) Representative: Serjeants LLP
(86) International application number: PCT/US2013/077803
(87) International publication number: WO 2014/105951

(56) References cited:
- EP-A2- 0 126 390
- WO-A1-87/05208
- US-A- 3 608 550
- US-A- 3 608 550
- US-A- 5 169 602
- US-A- 5 169 602
- US-A- 5 882 601
- US-B1- 6 269 976
- US-B2- 7 082 848

## Description

### Field of the Invention

The present invention relates to the field of fluid dispensing. More specifically, the present invention is directed to a needle kit for dispensing a product fluid.

### Background of the Invention

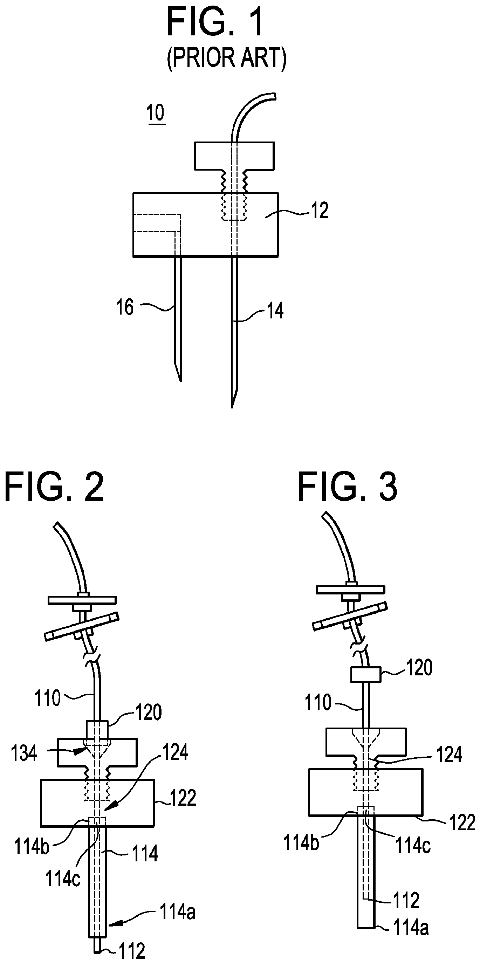

The dispensement of pharmaceutical fluids into a final dispense container is typically performed in either of two ways. First, the fluid may be dispensed in an 'open fill' in which the fluid is directly dispensed into the open mouth of an un-stoppered container which is subsequently stoppered and terminally sterilized. Second, the fluid may be dispensed in a trans-septal fill in which the fluid is dispensed through the septum, or stopper, that has already been attached to or across the open mouth of the container. Trans-septal filling also offers the possibility of asceptic dispensing in that a previously-sterilized stopped container may be dispensed into without subsequent terminal sterilization, provided certain asceptic conditions are met before and during the filling process. Open fills can provide a dispense lumen, such as a blunt cannula or a blunt needle, in registry with the open mouth of the container. The open fill dispense lumen may be a simple blunt cannula as no piercing of a septum is required. However, open fills may be performed asceptically by maintaining the fill environment at an environmentally clean level, such as one meeting Class 100 standards, and by including a pair of sterilizing filters in the dispense flowpath before the dispense lumen. As shown in Figure 1, trans-septal filling typically includes a device 10 including a block or fitting 12 supporting a sharpened fill needle 14 to be inserted through the septum of a dispense container so to provide the fluid to the dispense container and a sharpened vent needle 16 to be inserted through the septum to allow gas from the container to be displaced by the fluid. In both the open fill and trans-septal fill processes, the dispense cannula or fill needle are formed from metal. Prior to these metallic dispense cannulas or fill needles, the product fluid will typically flow through an elastomeric conduit as well as a fitting where the conduit is placed in fluid communication with the dispense cannula or needle.

The art lacks a dispense flowpath useful for either open fill or trans-septal fill that provides only a single material to contact the fluid through dispensing. The preamble of claims 1 and 5 is derivable from US 5,169,602.

### Summary of the Invention

The invention is defined by the appended claims.

The present invention provides a single concept which can satisfy the needs for an (1) an open fill, terminally sterilized, product, (2) a trans-septal fill, terminally sterilized, product, (3) an open fill, aseptic dispensed, product, and (4) a trans-septal fill, aseptic dispense, product. The aseptic applications may employ sterilizing filters on the dispense conduit of the present invention.

According to aspects of the present invention, for a trans-septal fill application, once a dispense needle or cannula reaches its final position prior to dispensing, with its distal tip extending through the septum of a product dispense container, an elongate inner tube is provided through the interior of needle or cannula so that one end of the distal end of inner tube extends to where it is even with or, desirably, beyond the distal end of the needle or cannula. The product fluid may then be directed through the inner tube and into the container, without contacting the material of the cannula or needle.

For open fill applications, the present invention contemplates that the inner tube may be directed through the cannula or needle at any time prior to dispensing the product fluid. That is, for open fill applications, the inner tube may be directed through the cannula or needle prior to the needle, carrying the tube with it, being positioned at a location at which filling may commence.

The present invention provides a method of dispensing a product fluid according to claim 1 and a needle kit for dispensing a product fluid according to claim 5.

### Brief Description of the Drawings

Figure 1 depicts a prior art trans-septal fill system in which a product fluid contacts surfaces made of multiple materials.
Figure 2 depicts a dispense system of the present invention as used in a terminal-sterilization open fill dispensing process.
Figure 3 depicts the dispense system of Figure 2 with the internal tube retracted into the cannula.
Figure 4 depicts a dispense system of the present invention as used in an asceptic open fill dispensing process.
Figure 5 depicts the dispense system of Figure 4 with the internal tube retracted into the cannula.
Figure 6 depicts a dispense system of the present invention as used in a trans-septal fill process.
Figure 7 depicts the dispense system of Figure 6 with the internal tube retracted into the cannula.
Figure 8 depicts another dispense system of the present invention as used in a trans-septal fill process.
Figure 9 depicts the dispense system of Figure 8 with the internal tube retracted into the cannula.

### Detailed Description of the Preferred Embodiments

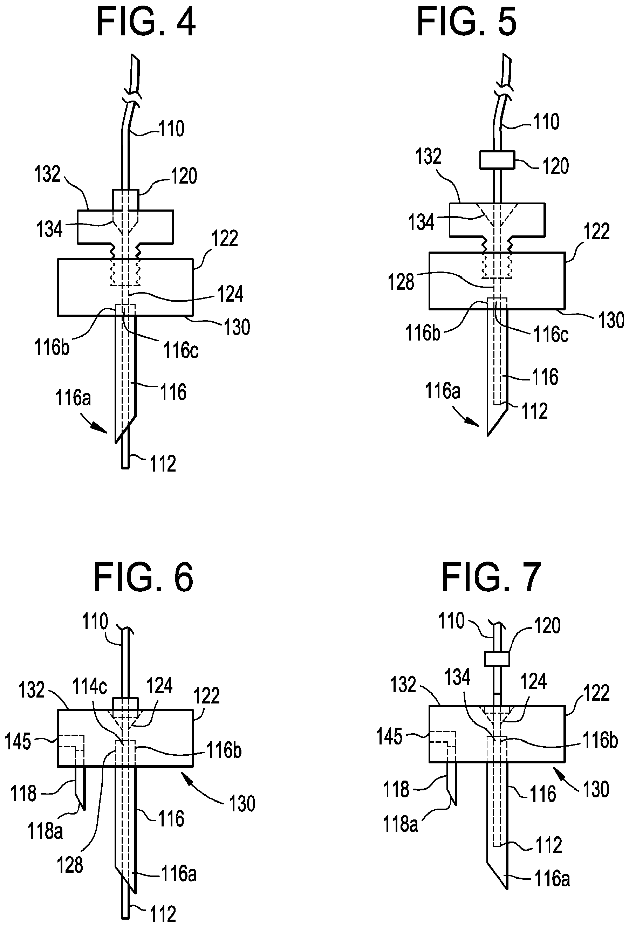

With reference to Figures 2-9, the present invention provides a method and devices for dispensing a product fluid so that the product fluid only contacts a single material throughout the dispensing operation. For trans-septal fill operations, the present invention provides an elongate polymeric conduit, or tube, 110 having a distal end 112 which is insertable through a dispense cannula 114 or needle 116 to extend to a position even with or past the distal end of the outer cannula 114a or needle 116a. The needle 116, intended to be inserted through a septum or stopper inserted into or across the mouth of the dispense container, typically includes a sharpened distal end 116a but may include a blunt distal end 116a as permitted by the design of the stopper. For open fill operations, the present invention provides an elongate polymeric conduit 110 having one end 112 which is insertable through a dispense cannula 114 or needle 116 to a position either in overlying registry with or within the open mouth of a container to be filled. The open fill needle may include a blunt distal tip 116a or may still include a sharpened distal tip 116a. By way of illustration and not of limitation, the elongate conduit of the present invention may be formed from a polymer such as, but not limited to, PTFE, although any suitable material for the fluid is contemplated for the present invention. Desirably, the conduit 110 of the present invention supports a ferrule 120 about one segment thereof which will act as a positive stop for insertion of the conduit but which ensures that the distal end 112 of the conduit extends past the plane of the dispense opening of the cannula or needle. The present invention further contemplates that the plane of the dispense opening of the distal end of the conduit will extend past the most distal portion of the dispense cannula or needle.

The present invention may be employed in both open fill and trans-septal fill applications as well as in terminally sterilized or asceptic dispense opeations. For open fill transfers, present invention contemplates positioning the dispense cannula 114 with its distal end 114a in registry with the open mouth of a dispense container. Alternatively, the distal end 114a of the dispense cannula 114 may be extended into the open mouth of the dispense container. In open fill transfers, the present invention provides for directing the polymeric conduit 110 of the present invention through the dispense cannula 114 to its dispense position either before, during, or after positioning of the dispense cannula in registry with or within the container mouth.

As shown in Figures 2-9, conduit 110 is movable from a first position (shown in Figures 3, 5, 7, 9) where its distal end 112 is positioned proximal the distal tip 114a or 116a of a cannula 114 or needle 116, respectively, to a second position (shown in Figures 2, 4, 6, 8) where distal end 112 of tube 110 extends even with or beyond distal tip 114a or 116a of cannula 114 or needle 116, respectively. The present invention further contemplates being able to retract tube 110 to the first position, such that tube 110 is movable between the first and second positions with respect to the cannula 114 or needle 116. The present invention then contemplates directing conduit 110 to a dispense position that is either co-extensive with the distal end 114a of the cannula 114 or that is beyond the distal end 114a of the cannula 114. The present invention further contemplates positioning a ferrule 120 about a portion of tube 110 and extending tube 110 through cannula 114 until ferrule 120 abuts a fitting 122 which supports the proximal end of 114b of cannula 114. The present invention also contemplates that other means for ensuring the proper positioning of tube 110 is employed such as, but not limited to, monitoring a spot on the outer surface of tube 110 or otherwise fixedly supporting tube 110 so as to travel with that support. Block, or fitting, 122 defines an elongate passageway 124 therethrough. Tube 110 is extended through passageway 124 and cannula 114 until ferrule 120 abuts block 122. Desirably, when ferrule 120 abuts either proximal end 114b of cannula 114 or block 122, distal end 112 of tube 110 will be in a dispense position that ensures no fluid dispensed from tube 110 will contact cannula 114.

For trans-septal transfers, the distal tip 116a of needle 116 is first extended through the septum or stopper placed within or across the mouth of the dispense container. The method then includes the step of pushing on block 122 to the septum, or to any position which ensures proper positioning of the distal tip 116a of needle 116 and distal tip 118a of vent needle 118. At this position, tube 110 may deliver fluid past tube end 112 into the container while vent needle 118 will allow gas to vent therethrough and out a vent passageway 145 defined by block 122. The method then includes the step of pushing on tube 110 or ferrule 120 so that tube end 112 now extends past the distal tip of needle 116. For both the open fill and trans-septal transfers, the present invention contemplates the provision of ferrule 120 positioned about the conduit 110 so as to be moved towards the dispense end 116a of needle 116, until it abuts against either the proximal end 116b of needle 116 or against a fitting 122 supporting needle 116.

The present invention contemplates that other methods or devices may be employed to limit the insertion of the conduit, such as, by way of illustration and not of limitation, an optical reader of an indicia on the outer surface of the conduit which provides for the generation of a stop signal to a device moving the conduit, abutment of a holding device which engages the conduit against a hard stop, or precise measurements so that an actuator only extends a fixed distance sufficient to move distal end 112 of the conduit 110 from a first position withdrawn from the distal end 114a or 116a of the cannula 114 or needle 116 to a second position where the distal end 112 of the conduit 110 extends even with or past the dispense opening defined by the distal end 114a or 116a of the cannula 114 or needle 116.

The present invention further contemplates that the proximal end of the conduit 110 may either extend to a bulk container of a fluid to be dispensed, or may connect to be placed in fluid communication with other devices used when dispensing the fluid. For example, the proximal end of the conduit may extend from the outlet port of a downstream valve of a dispense assembly such as is disclosed in Applicant's patent application WO 2014/105946 entitled "DUAL-FILTER DUAL-INTEGRITY TEST ASSEMBLY".

The fittings 122 of the present invention desirably support a proximal end 114b or 116b of the dispense cannula 114 or needle 116 so as to be positioned within a recess 128 opening on a first face 130 of the fitting 122 opposite to a second face 132 of the fitting 122 from which the distal end of the cannula or needle depends. The recess 128 of fitting 122 desirably provides a tapering surface 134 extending from the second face 132 towards the proximal end 114b or 116b of the cannula 114 or needle 116. The tapering surface 134 is desirably a conical surface tapering towards the proximal end of the cannula so as to assist the insertion of the distal end 112 of conduit 110 through fitting 122, into the proximal opening 114c or 116c defined by the proximal end 114b or 116b of the cannula 114 or needle 116, respectively.

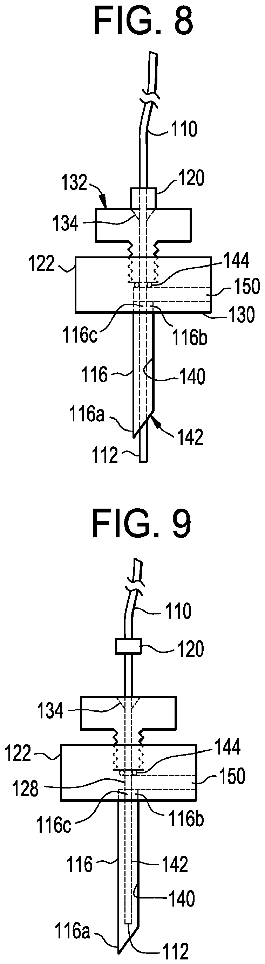

As shown in Figures 8 and 9, for asceptic transfer with only one spike or needle 116 through the septum, the present invention contemplates a method of running tube 110 through the ferrule 120, using the ferrule 120 to hold the tube in place so as to not transfer through anything except the PTFE/Teflon of the tube. The method then includes the step of pushing on the block to the septum (or to any position which ensures proper positioning of the distal tip 116a of needle 116), and then pushing on tube 110 or ferrule 120 so that tube end 112 now extends past the distal tip 116a of needle 116. Space 142 between tube 110 and the interior surface 140 of needle 116 allows venting out through open vent passage 150 defined by block 122. Vent passage 150 may be in direct fluid communication with recess 128 although it may be desirable to provide an elastomeric gasket 144 to seal a portion of recess 128 from vent passage 150 so as to further direct the outgassing.

With the dispense system, kits, and method of the present invention, a product fluid never sees any material other than the PTFE/tubing as it is dispensed into a dispense container, resulting in less chance of contamination of the product fluid.

While the present invention has been described as first moving a needle into place to a dispense position, the present invention alternatively contemplates that the present invention may be applied in a system which moves a container into position with a stationary dispense cannula or needle. In such a system, the septum or open mouth of the container could be pushed up to a block or fitting supporting the dispense cannula or needle, and then pushing both the container and fitting up until contact is made between the fitting and the ferrule about the conduit. Therefore, references to 'moving' or 'positioning' a cannula or needle of the present invention may include moving a dispense container so that the cannula or needle is in the dispense position. Alternatively still, the present invention contemplates that after the container is moved into position, the conduit may then be moved to the dispense position until the ferrule contacts the fitting.

Additionally, the present invention provides a fitting for a dispense cannula or needle which also supports a venting needle so as to allow gas to vent from a container through the vent needle and out the fitting as the product fluid is introduced into the container. Additionally, still, while the present invention contemplates that the conduit will substantially conform in size and shape to the interior surface of the dispense cannula or needle, the present invention further contemplates that the cannula or needle may be over-sized as compared to the conduit so as to allow venting back through the additional space in the cannula or needle and out through a vent passage in the fitting which also communicates with the cannula or needle. With an over-sized cannula or needle, the present invention further contemplates that the cannula or needle wall defines a vent port some spaced distance from the dispense end of the cannula or needle through which the venting gas may flow as product fluid enters the dispense container.

The conduit of the present invention may be provided as a disposable member which is used to dispense the contents from a single bulk container. The disposable member may be provided as part of a kit with a conduit and a dispense cannula or needle which the conduit is adaptable to be inserted through in accordance with the present invention.

While the particular embodiment of the present invention has been shown and described, it will be obvious to those skilled in the art that changes and modifications may be made without departing from the teachings of the invention. The matter set forth in the foregoing description and accompanying drawings is offered by way of illustration only and not as a limitation. The actual scope of the invention is intended to be defined in the following claims when viewed in their proper perspective based on the prior art.

## Claims

1. A method of dispensing a product fluid comprising the steps of:
a) directing the distal open end (114a, 116a) of an elongate needle (114, 116) to be in registry with the mouth of a container,
b) providing an elongate inner tube (110) through the interior of said needle (114, 116) so that one end (112) of said inner tube (110) extends beyond the distal end (114a, 116a) of said needle (114, 116); and
c) directing the product fluid through said inner tube (110) into the container;
wherein said providing step comprises the step of moving the inner tube (110) by a device from a first position wherein said one end (112) of said inner tube (110) resides within said needle (114, 116) to a second position wherein said one end (112) of said inner tube (110) extends beyond the distal end (114a, 116a) of said needle (114, 116), **characterized in that** said providing step further comprises optically reading by an optical reader an indicium on an outer surface of the inner tube (110) and generating by the optical reader a stop signal for the device moving the inner tube (110).

2. A method according to claim 1, wherein a proximal end (114b, 116b) of said needle (114, 116) is supported by a fitting (122).

3. A method according to claim 1 or claim 2, further comprising the step of directing the distal end (114a, 116a) of the needle (114, 116) through the mouth of the container.

4. A method according to claim 3, further comprising the step of directing the distal end (114a, 116a) of the needle (114, 116) through a septum positioned across or within the mouth of the container such that the distal end (114a, 116a) of the needle (114, 116) extends beyond the septum.

5. A method according to any preceding claim, wherein said inner tube (110) is made of a polymer, preferably PTFE.

6. A method according to claim 2, wherein said fitting (122) includes a fitting body defining opposed first and second openings, and an elongate needle passageway extending in fluid communication therebetween, said proximal end (114b, 116b) of said needle (114, 116) positioned adjacent to said first opening.

7. A method according to claim 6, wherein said first opening of said fitting (122) is further defined by a conical surface (134) tapering towards said proximal end (114b, 116b) of said needle (114, 116).

8. A method according to claim 7, wherein said proximal end (114b, 116b) of said needle (114, 116) is recessed within said fitting (122).

9. A method according to any preceding claim, wherein said distal end (116a) of said needle (116) includes a sharpened point.

10. A method according to any of claims 1 to 8, wherein said distal end (114a) of said needle (114) is substantially blunt.

11. A needle kit for dispensing a product fluid comprising:
a first elongate needle (114, 116) having opposed proximal (114b, 116b) and distal (114a, 116a) ends and an elongate needle body extending therebetween, said proximal end (114b, 116b) defining a first opening, said distal end defining a second opening and said body defining an elongate passageway extending in fluid communication between said first and second openings;
a fitting (122) supporting said proximal end (114b, 116b) of said first needle (114, 116);
an elongate inner tube (110) having opposed first and second (112) ends and an elongate tubular wall extending therebetween, said first end defining an inlet port, said second end (112) defining an outlet port and said wall defining an elongate fluidpath extending in fluid communication between said inlet port and said outlet port, said inner tube (110) extendable between a first position wherein said second end (112) does not extend through said passageway beyond said distal end (114a, 116a) of said first needle (114, 116) and a second position wherein said wall of said inner tube (110) extends through said passageway such that said second end (112) extends beyond said distal end (114a, 116a) of said first needle (114, 116);
an optical reader; and
a device arranged to move the inner tube (110) between the first and second positions;
**characterized in that** the optical reader is adapted to read an indicium on an outer surface of the inner tube (110) and to generate a stop signal for the device arranged to move the inner tube (110).

12. A needle kit according to claim 11, further comprising: a second needle (118) supported by said fitting, said second needle (118) including opposed proximal and distal (118a) ends and an elongate needle body extending therebetween, said proximal end defining a first aperture, said distal end (118a) defining a second aperture and said body defining an elongate passageway extending in fluid communication between said first and second apertures.

13. A needle kit according to claim 11 or claim 12 wherein said inner tube (110) is made of a polymer, preferably PTFE.

14. A needle kit according to any of claims 11 to 13, wherein said fitting (122) includes a fitting body defining opposed first and second openings, and an elongate needle passageway extending in fluid communication therebetween, said proximal end (114b, 116b) of said needle (114, 116) positioned adjacent to said first opening.

15. A needle kit according to claim 14, wherein said first opening of said fitting (122) is further defined by a conical surface (134) tapering towards said proximal end (114b, 116b) of said needle (114, 116).

16. A needle kit according to claim 15, wherein said proximal end (114b, 116b) of said needle (114, 116) is recessed within said fitting (122).

17. A needle kit according to any of claims 11-16 wherein said distal end (116a) of said needle (116) includes a sharpened point.

18. A needle kit according to any of claims 11-16, wherein said distal end (114a) of said needle (114) is substantially blunt.

## Patentansprüche

1. Verfahren zur Abgabe eines Produktfluids, umfassend die Schritte von:
a) Richten des distalen offenen Endes (114a, 116a) einer länglichen Nadel (114, 116), um deckungsgleich mit der Öffnung eines Behälters zu sein,
b) Bereitstellen eines länglichen Innenrohrs (110) durch das Innere der Nadel (114, 116), so dass sich ein Ende (112) des Innenrohrs (110) über das distale Ende (114a, 116a) der Nadel (114, 116) hinaus erstreckt; und
c) Richten des Produktfluids durch das Innenrohr (110) in den Behälter hinein;
wobei der Bereitstellungsschritt den Schritt des Bewegens des Innenrohrs (110) durch eine Vorrichtung von einer ersten Position, in der sich das eine Ende (112) des Innenrohrs (110) innerhalb der Nadel (114, 116) befindet, zu einer zweiten Position umfasst, in der sich das eine Ende (112) des Innenrohrs (110) über das distale Ende (114a, 116a) der Nadel (114, 116) hinaus erstreckt, **dadurch gekennzeichnet, dass** der Bereitstellungsschritt weiter das optische Lesen eines Indikators auf einer Außenfläche des Innenrohrs (110) durch einen optischen Leser und das Erzeugen eines Stoppsignals für die Vorrichtung, die das Innenrohr (110) bewegt, durch den optischen Leser umfasst.

2. Verfahren nach Anspruch 1, wobei ein proximales Ende (114b, 116b) der Nadel (114, 116) durch eine Halterung (122) getragen wird.

3. Verfahren nach Anspruch 1 oder Anspruch 2, weiter umfassend den Schritt des Richtens des distalen Endes (114a, 116a) der Nadel (114, 116) durch die Öffnung des Behälters.

4. Verfahren nach Anspruch 3, weiter umfassend den Schritt des Richtens des distalen Endes (114a, 116a) der Nadel (114, 116) durch eine Trennwand, die quer über oder innerhalb der Öffnung des Behälters angeordnet ist, so dass sich das distale Ende (114a, 116a) der Nadel (114, 116) über die Trennwand hinaus erstreckt.

5. Verfahren nach einem vorstehenden Anspruch, wobei das Innenrohr (110) aus einem Polymer, vorzugsweise PTFE, hergestellt ist.

6. Verfahren nach Anspruch 2, wobei die Halterung (122) einen Halterungskörper, der gegenüberliegende erste und zweite Öffnungen definiert, und einen länglichen Nadeldurchgang aufweist, der sich in Fluidverbindung dazwischen erstreckt, wobei das proximale Ende (114b, 116b) der Nadel (114, 116) benachbart zu der ersten Öffnung positioniert ist.

7. Verfahren nach Anspruch 6, wobei die erste Öffnung der Halterung (122) weiter durch eine konische Fläche (134) definiert ist, die sich zu dem proximalen Ende (114b, 116b) der Nadel (114, 116) hin verjüngt.

8. Verfahren nach Anspruch 7, wobei das proximale Ende (114b, 116b) der Nadel (114, 116) innerhalb der Halterung (122) vertieft ist.

9. Verfahren nach einem vorstehenden Anspruch, wobei das distale Ende (116a) der Nadel (116) eine geschärfte Spitze aufweist.

10. Verfahren nach einem der Ansprüche 1 bis 8, wobei das distale Ende (114a) der Nadel (114) im Wesentlichen stumpf ist.

11. Nadelkit zur Abgabe eines Produktfluids, umfassend:
eine erste längliche Nadel (114, 116) mit gegenüberliegenden proximalen (114b, 116b) und distalen (114a, 116a) Enden und einem länglichen Nadelkörper, der sich dazwischen erstreckt, wobei das proximale Ende (114b, 116b) eine erste Öffnung definiert, das distale Ende eine zweite Öffnung definiert und der Körper einen länglichen Durchgang definiert, der sich in Fluidverbindung zwischen den ersten und zweiten Öffnungen erstreckt;
eine Halterung (122), die das proximale Ende (114b, 116b) der ersten Nadel (114, 116) trägt;
ein längliches Innenrohr (110) mit gegenüberliegenden ersten und zweiten (112) Enden und einer länglichen, rohrförmigen Wand, die sich dazwischen erstreckt, wobei das erste Ende eine Einlassöffnung definiert, das zweite Ende (112) eine Auslassöffnung definiert und die Wand einen länglichen Fluidweg definiert, der sich in Fluidverbindung zwischen der Einlassöffnung und der Auslassöffnung erstreckt, wobei das Innenrohr (110) zwischen einer ersten Position, in der sich das zweite Ende (112) nicht durch den Durchgang über das distale Ende (114a, 116a) der ersten Nadel (114, 116) hinaus erstreckt, und einer zweiten Position ausfahrbar ist, in der sich die Wand des Innenrohrs (110) durch den Durchgang erstreckt, so dass sich das zweite Ende (112) über das distale Ende (114a, 116a) der ersten Nadel (114, 116) hinaus erstreckt;
einen optischen Leser; und
eine Vorrichtung, die angeordnet ist, um das Innenrohr (110) zwischen den ersten und zweiten Positionen zu bewegen;
**dadurch gekennzeichnet, dass** der optische Leser angepasst ist, um einen Indikator auf einer Außenfläche des Innenrohrs (110) zu lesen und ein Stoppsignal für die Vorrichtung zu erzeugen, die angeordnet ist, um das Innenrohr (110) zu bewegen.

12. Nadelkit nach Anspruch 11, weiter umfassend:
eine zweite Nadel (118), die durch die Halterung getragen wird, wobei die zweite Nadel (118) gegenüberliegende proximale und distale (118a) Enden und einen länglichen Nadelkörper aufweist, der sich dazwischen erstreckt, wobei das proximale Ende eine erste Öffnung definiert, das distale Ende (118a) eine zweite Öffnung definiert und der Körper einen länglichen Durchgang definiert, der sich in Fluidverbindung zwischen den ersten und zweiten Öffnungen erstreckt.

13. Nadelkit nach Anspruch 11 oder Anspruch 12, wobei das Innenrohr (110) aus einem Polymer, vorzugsweise PTFE, hergestellt ist.

14. Nadelkit nach einem der Ansprüche 11 bis 13, wobei die Halterung (122) einen Halterungskörper, der gegenüberliegende erste und zweite Öffnungen definiert, und einen länglichen Nadeldurchgang aufweist, der sich in Fluidverbindung dazwischen erstreckt, wobei das proximale Ende (114b, 116b) der Nadel (114, 116) benachbart zu der ersten Öffnung positioniert ist.

15. Nadelkit nach Anspruch 14, wobei die erste Öffnung der Halterung (122) weiter durch eine konische Fläche (134) definiert ist, die sich zu dem proximalen Ende (114b, 116b) der Nadel (114, 116) hin verjüngt.

16. Nadelkit nach Anspruch 15, wobei das proximale Ende (114b, 116b) der Nadel (114, 116) innerhalb der Halterung (122) vertieft ist.

17. Nadelkit nach einem der Ansprüche 11-16, wobei das distale Ende (116a) der Nadel (116) eine geschärfte Spitze aufweist.

18. Nadelkit nach einem der Ansprüche 11-16, wobei das distale Ende (114a) der Nadel (114) im Wesentlichen stumpf ist.

## Revendications

1. Procédé de distribution d'un produit fluide comprenant les étapes consistant à :
a) diriger l'extrémité ouverte distale (114a, 116a) d'une aiguille allongée (114, 116) de manière à ce qu'elle coïncide avec l'embouchure d'un conteneur,
b) fournir un tube interne allongé (110) à travers l'intérieur de ladite aiguille (114, 116) de telle sorte qu'une extrémité (112) dudit tube interne (110) s'étend au-delà de l'extrémité distale (114a, 116a) de ladite aiguille (114 116) ; et
c) diriger le produit fluide à travers ledit tube interne (110) jusque dans le conteneur;
dans lequel ladite étape de fourniture comprend l'étape consistant à déplacer le tube interne (110) à l'aide d'un dispositif depuis une première position dans laquelle ladite une extrémité (112) dudit tube interne (110) réside dans ladite aiguille (114, 116) vers une seconde position dans laquelle ladite une extrémité (112) dudit tube interne (110) s'étend au-delà de l'extrémité distale (114a, 116a) de ladite aiguille (114, 116), **caractérisé en ce que** ladite étape de fourniture comprend en outre les étapes consistant à lire de manière optique par l'intermédiaire d'un lecteur optique une indication sur une surface externe du tube interne (110) et à générer par l'intermédiaire du lecteur optique un signal d'arrêt pour le dispositif déplaçant le tube interne (110).

2. Procédé selon la revendication 1, dans lequel une extrémité proximale (114b, 116b) de ladite aiguille (114, 116) est supportée par un raccord (122).

3. Procédé selon la revendication 1 ou la revendication 2, comprenant en outre l'étape consistant à diriger l'extrémité distale (114a, 116a) de l'aiguille (114, 116) à travers l'embouchure du conteneur.

4. Procédé selon la revendication 3, comprenant en outre l'étape consistant à diriger l'extrémité distale (114a, 116a) de l'aiguille (114, 116) à travers un septum positionné à travers ou à l'intérieur de l'embouchure du conteneur de telle sorte que l'extrémité distale (114a, 116a) de l'aiguille (114, 116) s'étend au-delà du septum.

5. Procédé selon l'une quelconque des revendications précédentes, dans lequel ledit tube interne (110) est constitué d'un polymère, de préférence du PTFE.

6. Procédé selon la revendication 2, dans lequel ledit raccord (122) inclut un corps de raccord définissant des première et seconde ouvertures opposées, et un passage d'aiguille allongé s'étendant en communication fluidique entre les deux, ladite extrémité proximale (114b, 116b) de ladite aiguille (114, 116) positionnée de manière adjacente à ladite première ouverture.

7. Procédé selon la revendication 6, dans lequel ladite première ouverture dudit raccord (122) est en outre définie par une surface conique (134) s'effilant vers ladite extrémité proximale (114b, 116b) de ladite aiguille (114, 116).

8. Procédé selon la revendication 7, dans lequel ladite extrémité proximale (114b, 116b) de ladite aiguille (114, 116) est en retrait dans ledit raccord (122).

9. Procédé selon l'une quelconque des revendications précédentes, dans lequel ladite extrémité distale (116a) de ladite aiguille (116) inclut une pointe aiguisée.

10. Procédé selon l'une quelconque des revendications 1 à 8, dans lequel ladite extrémité distale (114a) de ladite aiguille (114) est sensiblement émoussée.

11. Kit d'aiguilles pour distribuer un produit fluide comprenant :
une première aiguille allongée (114, 116) présentant des extrémités proximale (114b, 116b) et distale (114a, 116a) opposées et un corps d'aiguille allongé s'étendant entre les deux, ladite extrémité proximale (114b, 116b) définissant une première ouverture, ladite extrémité distale définissant une seconde ouverture et ledit corps définissant un passage allongé s'étendant en communication fluidique entre lesdites première et seconde ouvertures ;
un raccord (122) supportant ladite extrémité proximale (114b, 116b) de ladite première aiguille (114, 116) ;
un tube interne allongé (110) présentant des première et seconde (112) extrémités opposées et une paroi tubulaire allongée s'étendant entre les deux, ladite première extrémité définissant un orifice d'entrée, ladite seconde extrémité (112) définissant un orifice de sortie et ladite paroi définissant un trajet de fluide allongé en communication fluidique entre ledit orifice d'entrée et ledit orifice de sortie, ledit tube interne (110) pouvant s'étendre entre une première position dans laquelle ladite seconde extrémité (112) ne s'étend pas à travers ledit passage au-delà de ladite extrémité distale (114a, 116a) de ladite première aiguille (114, 116) et une seconde position dans laquelle ladite paroi dudit tube interne (110) s'étend à travers ledit passage de telle sorte que ladite seconde extrémité (112) s'étend au-delà de ladite extrémité distale (114a, 116a) de ladite première aiguille (114, 116) ;
un lecteur optique ; et
un dispositif agencé pour déplacer le tube interne (110) entre les première et seconde positions ;
**caractérisé en ce que** le lecteur optique est apte à lire une indication sur une surface externe du tube interne (110) et à générer un signal d'arrêt pour le dispositif agencé pour déplacer le tube interne (110).

12. Kit d'aiguilles selon la revendication 11, comprenant en outre :
une seconde aiguille (118) supportée par ledit raccord, ladite seconde aiguille (118) incluant des extrémités proximale et distale opposées (118a) et un corps d'aiguille allongé s'étendant entre les deux, ladite extrémité proximale définissant une première ouverture, ladite extrémité distale (118a) définissant un une seconde ouverture et ledit corps définissant un passage allongé s'étendant en communication fluidique entre lesdites première et seconde ouvertures.

13. Kit d'aiguilles selon la revendication 11 ou la revendication 12, dans lequel ledit tube interne (110) est constitué d'un polymère, de préférence du PTFE.

14. Kit d'aiguilles selon l'une quelconque des revendications 11 à 13, dans lequel ledit raccord (122) inclut un corps de raccord définissant des première et seconde ouvertures opposées, et un passage d'aiguille allongé s'étendant en communication fluidique entre les deux, ladite extrémité proximale (114b, 116b) de ladite aiguille (114, 116) positionnée de manière adjacente à ladite première ouverture.

15. Kit d'aiguilles selon la revendication 14,
dans lequel ladite première ouverture dudit raccord (122) est en outre définie par une surface conique (134) s'effilant vers ladite extrémité proximale (114b, 116b) de ladite aiguille (114, 116).

16. Kit d'aiguilles selon la revendication 15,
dans lequel ladite extrémité proximale (114b, 116b) de ladite aiguille (114, 116) est en retrait dans ledit raccord (122).

17. Kit d'aiguilles selon l'une quelconque des revendications 11-16, dans lequel ladite extrémité distale (116a) de ladite aiguille (116) inclut une pointe aiguisée.

18. Kit d'aiguilles selon l'une quelconque des revendications 11-16, dans lequel ladite extrémité distale (114a) de ladite aiguille (114) est sensiblement émoussée.
